# EUROPEAN PATENT APPLICATION

(11) **EP 4 144 757 A1**
(43) Date of publication of application: **08.03.2023**
(21) Application number: 21797724.8
(22) Date of filing: 15.04.2021
(51) Int. Cl.: C07K 14/575, A61K 38/00, A61P 3/04

(54) **LONG-ACTING GLP-1 AND GLUCAGON RECEPTOR DUAL AGONIST**

(30) Priority: 29.04.2020 KR 20200052923
(71) Applicant: Dong-A ST Co., Ltd., Seoul 02587 (KR)
(72) Inventor: YANG, Jae-Sung, Seoul 06624 (KR); LEE, Kyung-Seok, Yongin-si Gyeonggi-do 17079 (KR); CHAE, Yu-Na, Yongin-si Gyeonggi-do 16807 (KR); KIM, Tae-Hyoung, Seoul 05501 (KR); JUNG, Ill-Hun, Anyang-si Gyeonggi-do 13994 (KR); RYU, Chae-Lim, Seongnam-si Gyeonggi-do 13499 (KR); LEE, Dae-Young, Seoul 06277 (KR)
(74) Representative: Ladendorf, Oliver
(86) International application number: PCT/KR2021/004747
(87) International publication number: WO 2021/221359

(57) **Abstract**

The present invention relates to a novel long-acting acylated oxyntomodulin peptide analogue having dual agonism on GLP-1 and glucagon receptors (dual GLP-1R/GlucagonR agonism) and to a pharmaceutical composition comprising the same, for the prevention and treatment of obesity and overweight, or non-insulin-dependent diabetes accompanied by obesity and overweight. The acylated oxyntomodulin peptide analog of the present invention has dual agonism for GLP-1/glucagon receptors and has an excellent increased *in vivo* half-life, and the pharmaceutical composition comprising the same is useful for the treatment of metabolic diseases such as obesity and diabetes.

## Description

### [TECHNICAL FIELD]

The present invention relates to acylated oxyntomodulin peptide analogs with the improved pharmacokinetic activity of GLP-1 and glucagon receptor dual agonist, and a pharmaceutical composition comprising the same for the prevention or treatment of obesity and overweight, or non-insulin-dependent diabetes accompanying the same.

### [BACKGROUND ART]

Metabolic diseases, or metabolic syndrome, are usually caused by abnormalities in the metabolism of glucose, fat, proteins, and others. The term usually refers to various diseases caused by abnormalities in glucose and fat metabolism, including cancer, diabetes, bone metabolism disorders, fatty liver, obesity, and cardiovascular disease. According to the 2001 report of the National Cholesterol Education Program (NCEP) of the United States and 2012 publications of International Diabetes Federation (IDF), diagnosis of metabolic syndrome requires the presence of three or more of the following five factors: (1) abdominal obesity indicated by a waist circumference of 102 cm (NCEP) or 94 cm (IDF) for males and 88 cm (NCEP) or 80 cm (IDF) for females; (2) hypertriglyceridemia indicated by triglyceride level of 150 mg/dL or above; (3) HDL cholesterol level at or lower than 40 mg/dL (male) or 50 mg/dL (female); (4) hypertension indicated by a blood pressure of 130/85 mmHg or higher; (5) a fasting glucose level of 110 mg/dL or higher.

According to the World Health Organization (WHO), the global prevalence of obesity has more than doubled between 1980 and 2014. In 2014, 39% of adults aged 18 years and older (38% of men and 40% of women) were overweight and 13% (11% of men, 15% of women) were obese. The root cause of obesity and overweight is an energy imbalance between caloric intake and expenditure, the causes of which include a diet with high fat content and high energy density and reduced physical activity due to the nature of modem work and lifestyle, changes in the modes of transportation, and increased urbanization.

Diabetes mellitus (or diabetes), which is one of the diseases linked to obesity, is also rapidly increasing in prevalence; 4.7% of adults aged 18 years or older had diabetes in 1980, compared to 8.5% in 2015. The prevalence rate of diabetes is increasing faster in middle- and low-income countries and is among leading cause of blindness, renal failure, cardiac arrest, and stroke.

Glucagon is a hormone produced by alpha cells of the pancreas. It works to raise the concentration of glucose by stimulating gluconeogenesis and promoting the breakdown of glycogen stored in liver. When liver-stored glycogen becomes depleted, glucagon stimulates liver and kidney to synthesize new glucose. It is also known to affect appetite suppression and breaking down of triglyceride storage into fatty acids, causing increased metabolism, thereby affecting body weight loss (Diabetes.co.uk. the global diabetes community, Anim Sci J. 2016;87(9): 1090-1098).

Glucagon-like peptide-1 (GLP-1), a glucagon derivative, is a peptide hormone which reduces blood glucose. GLP-1 is secreted by the L-cells in the small intestine after food intake and has a very short half-life of less than 2 minutes. It is reported that glucose increases the secretion of GLP-1, which induces insulin secretion by pancreatic beta cells, ultimately controlling blood glucose level and improving beta cell function. GLP-1 also suppresses the secretion of glucagon, inhibits gastric emptying, and reduces food intake (Physiol Rev. 2007;87(4): 1409-1439).

Novo Nordisk's liraglutide is human GLP-1 derivative which has been developed to treat type 2 diabetes and obesity and is to be injected once per day. Liraglutide is a long-acting GLP-1 receptor agonist that binds to the same receptor as endogenous GLP-1, stimulating insulin secretion, thereby modulating blood glucose level, reducing appetite, suppressing weight gain, and lowering triglycerides. It was marketed in the US and Europe under the names Victoza for type 2 diabetes and Saxenda for obesity (Expert Rev Cardiovasc Ther. 2015;13(7):753-767). Exenatide, lixisenatide, albiglutide, dulaglutide, and semaglutide also have been developed as antidiabetic drugs. However, these GLP-1 receptor agonists have reported side effects such as nausea, vomiting and decreased appetite, headache and constipation, and abdominal bloating (Korean J Med. 2014;87(1):9-13).

Oxyntomodulin is a peptide derived from proglucagon, a precursor of glucagon, and consists of a 37 amino-acid peptide comprising the complete 29 amino acid sequence of glucagon. Oxyntomodulin is known to be a dual agonist that can bind both to GLP-1 and glucagon receptors. In non-clinical studies, oxyntomodulin has been reported to result in reduced feed intake, weight loss, increased energy expenditure, and improved glucose metabolism (Diabetes. 2009;58(10):2258-2266). In clinical studies, oxyntomodulin showed body weight loss effects of 2.3 kg on average when administered subcutaneously for 4 weeks, 3 times per day, to overweight and obese patients (Diabetes. 2005;54:2390-2395), and it also showed significant insulin secretion and blood glucose reduction effects against placebo (Diabetes. 2013;62 (Suppl. 1):A48); in another clinical study, it was shown that continual administration of oxyntomodulin reduces energy intake without side effects such as vomiting or appetite stimulation (J Clin Endocrinol Metab. 2003;88:4696-4701). Oxyntomodulin's effectiveness at glycemic control, lowering of food intake, and satiety promotion have garnered interests in its potential as a new method of obesity treatment and glycemic control (Molecular metabolism. 2014;3:241-251).

However, because oxyntomodulin, like GLP-1, can be cleaved by dipeptidyl peptidase-IV (DPP-IV), it is unstable *in vivo* and has a very short *in vivo* half-life (J Biol Chem. 2003;278: 22418-22423).

Therefore, there are reports of studies being conducted on DPP-IV-resistant oxyntomodulin derivatives that can selectively bind to GLP-1 and glucagon receptors in a balanced way to sustain the pharmacological and therapeutic action of oxyntomodulin for a long period of time and to overcome the side effects of each hormonal peptide (Diabetes. 2009;58(10):2258-2266), and several companies including Merck, Zealand, Medimmune, and Hanmi Pharmaceutical, are working on developing lead compounds.

Korean Patent Application No. 2017-0103798 and Korean Patent Application No. 2018-0095717 disclose acylated oxyntomodulin peptide analogs having dual action on GLP-1 and glucagon receptors. Among the acylated oxyntomodulin peptide analogs described in the above-mentioned prior art, Compound 3 has superior potency (EC50<10pM) to the GLP-1 and glucagon receptors compared to the endogenous hormone oxyntomodulin, outstanding activity on the GLP-1 and glucagon receptors, and has a better weight loss effect than liraglutide, the control, in the 1-week *in vivo* weight loss evaluation in mice.

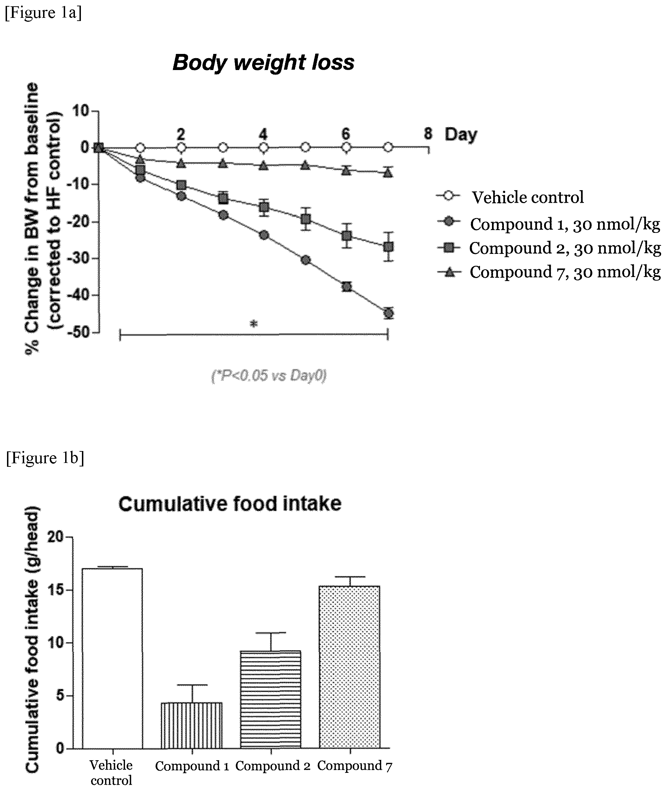

In the process of developing the above-mentioned Compound 3 for commercialization, the present inventors conducted pharmacokinetic evaluation in mice and found that the Compound's half-life was 3.3 hours, similar to that of Medimmune's MEDI0382 of 4.0 hours (Table 6, Figure 1). MEDI0382 is currently under clinical studies in the form of a once-a-day injection. Compound 3 may be developed as a once-a-day injection formulation, considering its half-life comparable to that of MEDI0382. However, once-daily formulations of a dual agonist of GLP-1 and glucagon receptors have the disadvantages requiring to be administered by injection every day.

Therefore, the present inventors recognized the need for the development of a long-acting acylated oxyntomodulin peptide analog that can be administered as an injection once a week to increase convenience by increasing the *in vivo* half-life while maintaining drug efficacy.

In efforts to develop a long-acting dual agonist of GLP-1 and glucagon receptors that meets the above needs, the present inventors updated Compound 3, an acylated oxyntomodulin peptide analog disclosed in Korean Patent Application No. 2018-0095717, to develop a long-acting acylated oxyntomodulin peptide analog that has dual GLP-1R/GlucagonR agonism and a long *in vivo* half-life by improving metabolic stability, and thus able to be injected once per week as an injection, culminating in the completion of the present invention.

### [DETAILED DESCRIPTION OF THE INVENTION]

### [TECHNICAL PROBLEM]

An object of the present invention is to provide an acylated oxyntomodulin peptide analog having dual agonism for GLP-1 and glucagon receptors with increased *in vivo* half-life. Another object of the present invention is to provide a pharmaceutical composition for preventing or treating a condition caused or characterized by obesity and overweight, or non-insulin dependent diabetes mellitus accompanied by obesity or overweight, comprising the acylated oxyntomodulin peptide analog. Another object is to provide an injectable formulation comprising the acylated oxyntomodulin peptide analog as an active ingredient that can be administered once a week.

### [TECHNICAL SOLUTION]

As a solution to the above-mentioned problem, the present invention provides a novel acylated oxyntomodulin peptide analog of the following Chemical Formula I:

<Chemical Formula I> His-Aib-Gln-Gly-Thr-Phe-Thr-Ser-Asp-X₁-Ser-Lys-Tyr-Leu-Asp-Aib-Arg-Arg-Ala-Gln-Asp-Phe-Val-Gln-Trp-Leu-Met-Asn-Thr-Lys-Glu-Tyr-Glu-X₂-Glu-Tyr-Glu (SEQ ID NO. 14)

In the formula above,
X₁ is a functionalized Lys, with [(2-(2-(2-aminoethoxy)ethoxy)acetoyl)₂]-[gamma glutamyl]-[octadecanoyl] conjugated to its side chain;
X₂ is a functionalized Lys with a lipophilic lipid or spacer-lipophilic lipid or polymeric moiety-spacer-lipophilic lipid or spacer-polymeric moiety-spacer-lipophilic lipid conjugated to its side chain;
the lipophilic lipid is of the following Structural Formula (1) or (2) below: 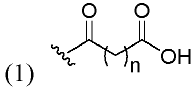 n = 12, 14 or 16 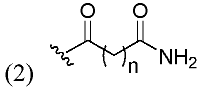 n = 16;
the polymeric moiety is 1-3 (one or two or three) of 2-(2-(2-aminoethoxy)ethoxy)acetoyl; and
the spacer is r-Glu or Lys.

In the present invention, the following three-letter and/or single-letter abbreviations are used to refer to amino acids:
Ala (A), Lys (K), Asn (N), Asp (D), Cys (C), His (H), Ile (I), Met (M), Ser (S), Val (V), Gly (G), Leu (L), Pro (P), Thr (T), Phe (F), Arg (R), Tyr (Y), Trp (W), Glu (E), Gln (Q), Aib (aminoisobutyric acid).

As used herein, the term "oxyntomodulin" refers to a peptide made from pre-glucagon, a precursor of glucagon, and the wild type oxyntomodulin has the amino acid sequence of HSQGTFTSDYSKYLDSRRAQDFVQWLMNTKRNRNNIA (SEQ ID NO.1).

In the lipophilic lipid, which is one of the components of X₂, the acyl group at the lipid terminal may be attached to the amine group in the Lys side chain or in the spacer via an amide bond; and comprises C14-C18 saturated hydrocarbon chain if the terminal carbon of the hydrocarbon chain is a carboxylic acid, or C18 saturated hydrocarbon chain if the terminal carbon of the hydrocarbon chain is in the form of an aminocarboxylic acid.

The spacer is r-Glu or Lys, and the amine group of the amino acid residue of Lys, or an α-amino group of r-Glu, may be covalently bonded to a lipophilic lipid or a polymeric moiety.

Though the present invention is not to be construed as limited in interpretation by a particular theory, it is believed that the acylation of X₁ of the above-mentioned Chemical Formula I stabilizes the alpha-helical structure of the peptide, increasing its pharmacological efficacy or selectivity at the GLP-1 receptor or/and the glucagon receptor (ACS Chem Biol. 2016;11:324-328), and the seven amino acid termini including X₂ (EYEX₂ EYE) is believed to bind to albumin in the bloodstream, thereby preventing the compound of the present invention from reacting as a substrate for various degrading enzymes in the bloodstream, and thus improving the half-life *in vivo* (Nat. Commun. 2017;8:16092).

Peptide embodiments of Chemical Formula I are Compound 1 (SEQ ID NO. 2), Compound 2 (SEQ ID NO. 3), Compound 3 (SEQ ID NO. 4), Compound 4 (SEQ ID NO. 5), Compound 5 (SEQ ID NO. 6), Compound 6 (SEQ ID NO. 7), and Compound 7 (SEQ ID NO. 8).

The acylated oxyntomodulin peptide analog of the present invention may be provided as an acid addition salt of any amine group present in its structure, a carboxylate salt of any carboxy group, or an alkali addition salt thereof.

In addition, the present invention relates to a pharmaceutical composition for the prevention and treatment of obesity and overweight, or non-insulin-dependent diabetes mellitus accompanied by obesity or overweight, comprising the above-mentioned acylated oxyntomodulin peptide analog as an active ingredient and a pharmaceutically acceptable excipient.

As used herein, the term "prevention" refers to any action that suppresses or delays the onset of a target disease. The term "treatment" refers to any action that mitigates, improves, or alleviates the symptoms of a condition or a disease that has developed.

Since the acylated oxyntomodulin peptide analog of the present invention is a dual agonist for the glucagon receptor and the GLP-1 receptor, it exhibits both the effects of GLP-1 on food intake and the effects of glucagon on fat metabolism and energy expenditure. Therefore, a pharmaceutical composition for the prevention and treatment of obesity and overweight, comprising the acylated oxyntomodulin peptide analog of the present invention, can induce medically beneficial effects in weight control through the combined effect of removing excessively accumulated fat and suppressing food intake.

In addition, the pharmaceutical composition comprising the acylated oxyntomodulin peptide analog of the present invention can be used for preventing or treating diabetes mellitus accompanying obesity or overweight by reducing blood glucose. In particular, it can be used to treat type 2 diabetes, which is non-insulin-dependent diabetes mellitus accompanying obesity. Although no limitation of the scope of interpretation to a particular theory is intended, the pharmaceutical composition comprising the acylated oxyntomodulin peptide analog of the present invention is a glucagon derivative and is highly active on the GLP-1 receptor, which lowers blood glucose, and thus is useful for glycemic control.

Therefore, the pharmaceutical composition comprising the acylated oxyntomodulin peptide analog of the present invention can be administered either alone or in combination with other related agents as part of a direct or indirect treatment of any condition caused or characterized by overweight, including treatment and prevention of obesity, morbid obesity, preoperative morbid obesity, obesity-related inflammation, obesity-related gallbladder disease, obesity-induced sleep apnea, and diabetes accompanied by obesity. In addition, the pharmaceutical composition comprising the acylated oxyntomodulin peptide analog of the present invention can be administered alone or in combination with other related agents to prevent conditions that may result from or may be related to the effect of body weight, such as metabolic syndrome, hypertension, arteriosclerosis-inducing dyslipidemia, atherosclerosis, arteriosclerosis, coronary heart disease, or stroke.

In addition, the present invention provides an injectable formulation comprising an acylated oxyntomodulin peptide analog as an active ingredient that can be administered once a week.

The injection formulation comprising the acylated oxyntomodulin peptide analog of the present invention can be used in combination with buffers, preservatives, analgesics, solubilizers, isotonic agents, stabilizers, and the like; and it may be prepared in unit dosage ampoules or multiple dosage forms.

The acylated oxyntomodulin peptide analog of the present invention exhibits improved pharmacokinetics (*in vivo* half-life) than the acylated oxyntomodulin peptide analog described in Korean Patent Application No. 2018-0095717. The improved *in vivo* half-life in the present invention can increase the convenience of use as a long-acting peptide analog such as a once-weekly injection that overcomes the disadvantages of the once-a-day injection.

Furthermore, "administration" used herein includes introducing a substance for therapeutic use to a patient by an appropriate method, and the pharmaceutical composition comprising the acylated oxyntomodulin peptide analog of the present invention may be administered through various routes and in various forms as long as the desired efficacy can be obtained by allowing the drug to reach the target tissue. That is, in addition to intraperitoneal administration, intravenous administration, and intramuscular administration, possible routes of administration also include subcutaneous administration, intradermal administration, oral administration, topical administration, intranasal administration, intrapulmonary administration, and rectal administration, though the formulation and administration method are not particularly limited.

The pharmaceutical composition comprising the acylated oxyntomodulin peptide analog of the present invention may comprise a pharmaceutically acceptable carrier, including but not limited to: binders, lubricants, disintegrants, excipients, solubilizers, dispersants, stabilizers, suspending agents, dyes, fragrances, etc. for oral administration; and various bases, excipients, lubricants, preservatives, etc. for topical administration. Examples of carriers, excipients and diluents include lactose, dextrose, sucrose, sorbitol, mannitol, xylitol, erythritol, maltitol, starch, acacia, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate or mineral oil.

The pharmaceutical composition comprising the acylated oxyntomodulin peptide analog of the present invention can be prepared in various ways by mixing with the above-mentioned carrier in addition to the injection formulation. For example, in the case of oral administration, it can be prepared in the form of tablets, troches, capsules, elixirs, suspensions, syrups, wafers, etc., and also formulated as solutions, suspensions, tablets, pills, capsules, sustained-release preparations, etc.

The dosage range according to the present invention varies depending on factors such as the patient's weight, age, sex, health condition, diet, excretion rate and the severity of the condition. For an adult patient, appropriate dosage may be 0.001 to 500 mg/kg per day.

### [ADVANTAGEOUS EFFECTS OF THE INVENTION]

The present invention provides a long-acting acylated oxyntomodulin peptide analog having activity against both GLP-1 and glucagon receptors and having an enhanced *in vivo* half-life. In particular, the acylated oxyntomodulin peptide analogs according to the present invention have a very outstanding *in vivo* half-life compared to not only wild type oxyntomodulin but also conventional acylated oxyntomodulin peptide analogs (Korean Patent Application No. 10-2018-0095717, acylated oxyntomodulin peptide analog).

Therefore, the present invention comprising a long-acting acylated oxyntomodulin peptide analog with an increased *in vivo* half-life, which can be administered once a week, overcomes the disadvantage of the existing formulations that require daily injection, thereby increasing convenience of use. In addition, it can be usefully applied primarily for the prevention or treatment of conditions caused or characterized by obesity or overweight, and further for the prevention or treatment of non-insulin-dependent diabetes mellitus accompanying obesity or overweight.

### [BRIEF DESCRIPTION OF THE DRAWINGS]

Figure 1 is a graph showing the results of body weight loss efficacy evaluation in mice after one week of repeated injection of Compound 1, Compound 2, or Compound 7 respectively, which are acylated oxyntomodulin peptide analogs according to the present invention. Figure 1a shows the weight loss result, and Figure 1b is a graph showing the result of cumulative feed intake.
Figure 2 is a graph showing the results of body weight loss drug efficacy evaluation in mice after one week of repeated injection of Compound 3, which is an acylated oxyntomodulin peptide analog according to the present invention. Figure 2a shows the weight loss result, and Figure 2b is a graph showing the result of cumulative feed intake.
Figure 3 is a graph showing the results of body weight loss efficacy evaluation in mice after one week of repeated injection of Compound 4, Compound 5, or Compound 6 respectively, which are acylated oxyntomodulin peptide analogs according to the present invention. Figure 3a shows the weight loss result, and Figure 3b is a graph showing the result of cumulative feed intake.
Figure 4 is a graph showing the pharmacokinetic results after a single-dose administration in mice of the acylated oxyntomodulin peptide analog according to the present invention.
Figure 5 is a graph showing the pharmacokinetic results after a single-dose administration in monkeys of the acylated oxyntomodulin peptide analog according to the present invention.
Figure 6 is a graph showing the results of the glucose tolerance improvement assay in mice of the acylated oxyntomodulin peptide analog according to the present invention.

### [DESCRIPTION OF EMBODIMENTS]

The present invention is further described in detail by reference to the following examples and experimental examples. These examples are provided for purposes of illustration only, to help a person skilled in the art understand the invention, and should not in any way be construed as limiting the scope of the present invention.

### <Example 1> Synthesis of acylated oxyntomodulin peptide analogs according to the present invention

The peptides and canonical peptide sequences comprising some amino acids of the present invention can be synthesized or purchased from commercial peptide manufacturers such as American Peptide Company or Bachem in the United States, or Anygen in Korea.

The acylated oxyntomodulin peptide analog in the present invention were synthesized using the Symphony X (synthesis scale: 0.1mmol) model of Protein Technologies, Inc. The structures of Compound 1 (SEQ ID NO. 2) through Compound 7 (SEQ ID NO. 8), which are acylated oxyntomodulin peptide analogs synthesized according to the present invention, are shown in Tables 1 and 2. The specific synthesis process is as follows:
A mixture of Fmoc-AA-OH (1 mmol), HBTU (1 mmol), NMM (n-methylmorpholine) (2 mmol) and DMF (7 ml) was placed in resin from which Fmoc has been removed and stirred at room temperature for 1 hour. The reaction solution drained and washed twice with 7ml of DMF (N,N-Dimethylmethanamide). Fmoc cleavage reaction was performed twice for 5 minutes at room temperature with 20% piperidine DMF solution (5ml), and the solution was washed 6 times with DMF (7ml). This process was repeated using an automated synthesizer to couple the amino acids.

For the Lys side synthesis, the coupling performed using Fmoc-K(dde)-OH or Fmoc-K(Alloc)-OH, and the last His coupled using Boc-His(trt)-OH. The synthesis of functionalized Lys side chains carried out using 2% hydralazine or tetrakis(triphenylphosphine)palladium(0) to remove the protected dde or Alloc, and then the desired side chain moieties (PEG2, rE, K, C14-18 di-acid, etc.) are coupled.

To 0.1 mmol of the peptide-resin obtained above, 8 ml of a solution of Reagent K (trifluoroacetic acid, water, thioanisole, 1,2-ethhandhiol (87.5, 5.0, 5.0, 2.5)) was added after cooling to 5-10°C. combined filtrate 100ml of diethyl ether for crystallization. The solid filtered he crude peptide was purified by preparative HPLC to obtain the target compound.

ShimadzuAxima Assurance MALDI-TOF was used for molecular weight analysis, with α-Cyano-4-hydroxycinnamic acid (CHCA) used as a matrix.

**[Table 1] Structure of acylated oxyntomodulin peptide analogs of the present invention**

| **Compound** | **Structure** |
|---|---|
| Compound 1 | 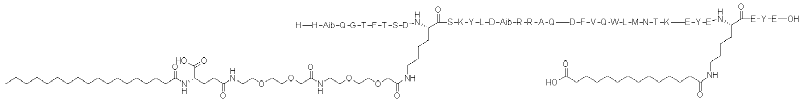 |
| Compound 2 | 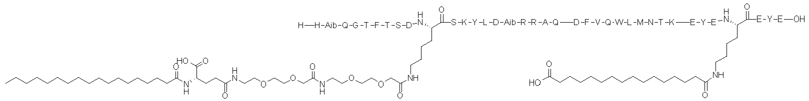 |
| Compound 3 | 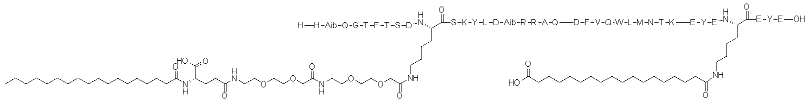 |
| Compound 4 | 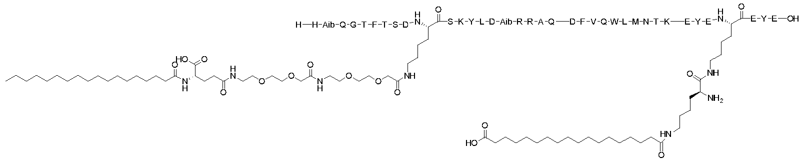 |
| Compound 5 | 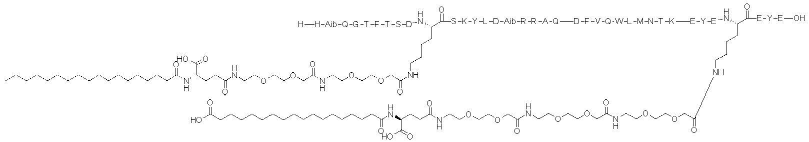 |
| Compound 6 | 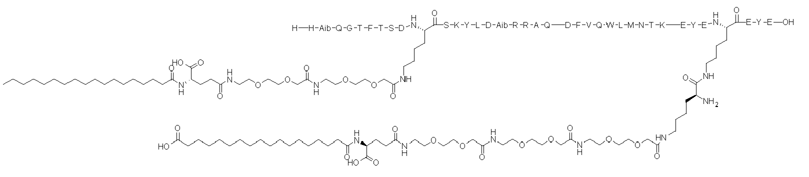 |
| Compound 7 | 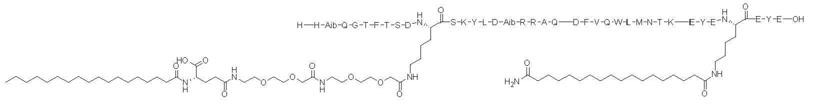 |

**[Table 2] Sequence and structure of acylated oxyntomodulin peptide analogs of the present invention**

| **SEQ ID NO.** | **Name** | **Sequence** |
|---|---|---|
| 1 | OXM (original) | H-HSQGTFTSDKSKYLDSRRAQDFVQWLMNTKRNRNNIA-OH |
| 2 | Compound 1 | H-HAibQGTFTSDK([(2-(2-(2-aminoethoxy)ethoxy)acetoyl)2]-[gammaglutamyl]-[octade canoyl])SKYLDAibRRAQDFVQWLMNTKEYEK(13-carboxy-1-oxotridecyl)EYE-OH |
| 3 | Compound 2 | H-HAibQGTFTSDK([(2-(2-(2-aminoethoxy)ethoxy)acetoyl)2]-[gammaglutamyl]-[octade canoyl])SKYLDAibRRAQDFVQWLMNTKEYEK(15-carboxy-1-oxopentadecyl)EYE-OH |
| 4 | Compound 3 | H-HAibQGTFTSDK([(2-(2-(2-aminoethoxy)ethoxy)acetoyl)2]-[gammaglutamyl]-[octade canoyl])SKYLDAibRRAQDFVQWLMNTKEYEK(17-carboxy-1-oxoheptadecyl)EYE-OH |
| 5 | Compound 4 | H-HAibQGTFTSDK([(2-(2-(2-aminoethoxy)ethoxy)acetoyl)₂]-[gammaglutamyl]-[octade canoyl])SKYLDAibRRAQDFVQWLMNTKEYEK([lysyl]-[17-carboxy-1-oxoheptadecyl])EYE- OH |
| 6 | Compound 5 | H-HAibQGTFTSDK([(2-(2-(2-aminoethoxy)ethoxy)acetoyl)₂]-[gammaglutamyl]-[octade canoyl])SKYLDAibRRAQDFVQWLMNTKEYEK([(2-(2-(2-aminoethoxy)ethoxy)acetoyl)₃]-[ gammaglutamyl]-[17-carboxy-1-oxoheptadecyl])EYE-OH |
| 7 | Compound 6 | H-HAibQGTFTSDK([(2-(2-(2-aminoethoxy)ethoxy)acetoyl)₂]-[gammaglutamyl]-[octade canoyl])SKYLDAibRRAQDFVQWLMNTKEYEK([lysyl]-[(2-(2-(2-aminoethoxy)ethoxy)acet oyl)₃]-[gammaglutamyl]-[17-carboxy-1-oxoheptadecyl])EYE-OH |
| 8 | Compound 7 | H-HAibQGTFTSDK([(2-(2-(2-aminoethoxy)ethoxy)acetoyl)₂]-[gammaglutamyl]-[octade canoyl])SKYLDAibRRAQDFVQWLMNTKEYEK(17-aminocarboxy-1-oxoheptadecyl)EYE-O H |

### <Comparative Example 1> Synthesis of acylated oxyntomodulin peptide analogs

For comparison with the present invention, an acylated oxyntomodulin peptide analog having structural similarity was synthesized by the method of Example 1. The following Comparative Example Compound 8 is described in Korean Patent Application No. 2018-0095717 as Compound 3, and it is a peptide analog that does not have the 7 amino acids (EYEX2EYE) at the C-terminal. Comparative Example Compound 9 is a peptide analog that is Comparative Example Compound 8 with a ligand mentioned in the literature (ACS Chem Biol. 2016;11:324-328) added. Comparative Example Compound 10 and Comparative Example Compound 11 are peptide analogs having an aminocarboxyl group at the carbon chain end of C14 and C16, respectively, as the lipophilic lipid in X₂. Comparative Example Compound 12 is a peptide analog in which the C16 is linked with a polymeric moiety as the lipophilic lipid in X₂. The structures of Comparative Example Compound 8 to Comparative Example Compound 12 are shown in Tables 3 and 4.

**[Table 3] Structure of acylated oxyntomodulin peptide analogs**

| **Compound** | **Structure** |
|---|---|
| Compound 8* | 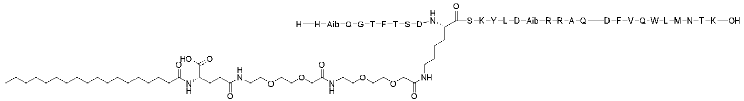 |
| Compound 9 | 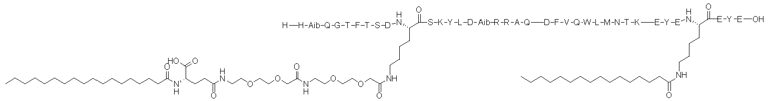 |
| Compound 10 | 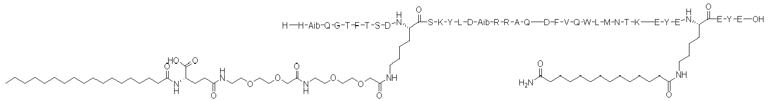 |
| Compound 11 | 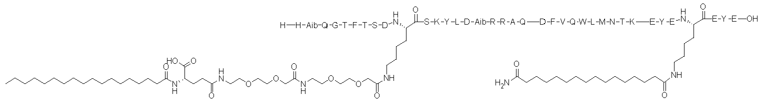 |
| Compound 12 | 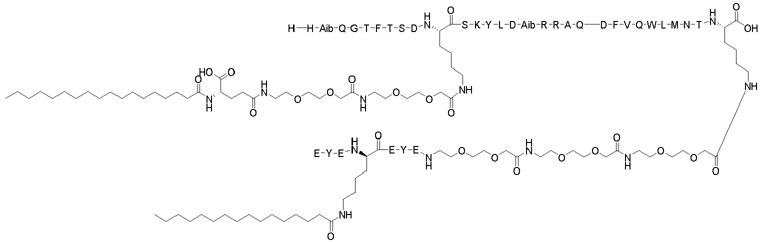 |

**[Table 4] Sequence and structure of acylated oxyntomodulin peptide analogs**

| **SEQ ID NO.** | **Name** | **Sequence** |
|---|---|---|
| 9 | Compound 8 | H-HAibQGTFTSDK([(2-(2-(2-aminoethoxy)ethoxy)acetoyl)2]-[gammaglutamyl]-[oct adecanoyl])SKYLDAibRRAQDFVQWLMNTK-OH |
| 10 | Compound 9 | H-HAibQGTFTSDK([(2-(2-(2-aminoethoxy)ethoxy)acetoyl)2]-[gammaglutamyl]-[oct adecanoyl])SKYLDAibRRAQDFVQWLMNTKEYEK(hexadecanoyl)EYE-OH |
| 11 | Compound 10 | H-HAibQGTFTSDK([(2-(2-(2-aminoethoxy)ethoxy)acetoyl)2]-[gammaglutamyl]-[oct adecanoyl])SKYLDAibRRAQDFVQWLMNTKEYEK(13-aminocarboxy-1-oxotridecyl)EY E-OH |
| 12 | Compound 11 | H-HAibQGTFTSDK([(2-(2-(2-aminoethoxy)ethoxy)acetoyl)2]-[gammaglutamyl]-[oct adecanoyl])SKYLDAibRRAQDFVQWLMNTKEYEK(15-aminocarboxy-1-oxopentadecyl )EYE-OH |
| 13 | Compound 12 | H-HAibQGTFTSDK([(2-(2-(2-aminoethoxy)ethoxy)acetoyl)2]-[gammaglutamyl]-[oct adecanoyl])SKYLDAibRRAQDFVQWLMNTKEYEK([(2-(2-(2-aminoethoxy)ethoxy)acet oyl)3]-[hexadecanoyl])EYE-OH |

### <Experimental Example 1> GLP-1 and glucagon receptor activation assay

Human GLP-1 or glucagon receptors were transiently overexpressed in cells, so that the acylated oxyntomodulin peptide analog of the present invention could activate the receptors resulting in a rise in cyclic adenosine monophosphate (cAMP), which successively activates cyclic adenosine monophosphate response elements (CRE). Then, the resulting increased luciferase activity was evaluated as a measurement of the effect on each receptor activation.

For positive control, endogenous ligand GLP-1 or glucagon was used for respective evaluation. The above-mentioned acylated oxyntomodulin peptide analogs of Comparative Example 8-12 were synthesized and used as comparative examples.

Human GLP-1 or glucagon expression vector ("OriGene") was transiently transfected into Chinese hamster ovary cells (CHO-K1), with plasmid DNAs that can induce expression of firefly luciferase or *Renilla* luciferase, using Lipofectamine Plus Reagent (Invitrogen). After 3 hours of transfection, medium was exchanged to Alpha Minimal Essential Medium (α-MEM) comprising 10% fetal bovine serum (FBS). Next day, the medium was exchanged to α-MEM comprising the acylated oxyntomodulin peptide analog of the present invention and 0.1% bovine serum albumin (BSA). After 6 hours, dual luciferase assay reagent (Promega) was added in the same amount as the medium in which the cells were submerged, and firefly luciferase and *Renilla* luciferase activities were successively measured. Firefly luciferase activity values were corrected against *Renilla* luciferase activity to yield transfection efficiency.

To measure the receptor activation efficacy, multi-concentration test was performed on the acylated oxyntomodulin peptide analog of the present invention to obtain the relative activation (%) of the maximum effect of the analog on either GLP-1 or glucagon, and the concentration indicating 50% activation (EC50) was calculated using non-linear regression analysis. The resulting values are shown in Table 5.

**[Table 5] Human GLP-1/glucagon receptor activation ability of acylated oxyntomodulin peptide analogs**

| | **Compound** | **Receptor activation** | | | |
|---|---|---|---|---|---|
| | | **GLP-1 receptor** | | **GCG receptor** | |
| | | **EC50^{∗}** | **Maximum activation (% vs GLP-1)** | **EC50^{∗}** | **Maximum activation (% vs GCG)** |
| Example | Compound 1 | A | 101.7% | A | 107.0% |
| | Compound 2 | B | 103.9% | B | 100.1% |
| | Compound 3 | B | 98.3% | B | 98.3% |
| | Compound 4 | B | 87.9% | C | 95.4% |
| | Compound 5 | B | 93.6% | B | 99.4% |
| | Compound 6 | B | 83.8% | B | 89.1% |
| | Compound 7 | A | 90.8% | A | 100.1% |
| Comparative Example | Compound 8 | A | 99.3% | A | 102.8% |
| | Compound 9 | B | 70.4% | B | 73.3% |
| | Compound 10 | A | 105.3% | A | 110.5% |
| | Compound 11 | A | 92.8% | A | 98.5% |
| | Compound 12 | A | 101.9% | A | 89.6% |

| | | | | | |
|---|---|---|---|---|---|
| ^{∗} A: EC₅₀<100pM, B: 100pM≤EC₅₀<1000pM, C: EC₅₀≥1000pM | | | | | |

Experimental results show that the compounds of the acylated oxyntomodulin peptide analogs according to the present invention exhibit dual agonism against GLP-1 and glucagon receptors like wild type oxyntomodulin, and exhibit sufficient activity comparable to the endogenous hormones GLP-1 and glucagon in terms of maximum efficacy.

### <Experimental Example 2> Body weight loss efficacy evaluation by 1-week repeated injection of the oxyntomodulin peptide analog of the present invention

This experiment was conducted to confirm the weight loss efficacy of the acylated oxyntomodulin peptide analog according to the present invention. Male laboratory mice (C57BL/6 mouse) were provided with diet containing high fat. The mice with high-fat-diet-induced obesity were separated into groups by body weight before the experiment began. Compound 1, Compound 2, and Compound 7, which are examples according to the present invention, were each prepared in sterile distilled water for injection containing 0.1% Cremophor EL to a dosage of 30 nmol/kg. They were injected subcutaneously once a day for a total of 7 days as described in Table 6. Body weight and food intake was measured once a day, at the same time each day, to evaluate the body weight loss efficacy of the acylated oxyntomodulin analogs compared to the initial body weight. The results are shown in Figures 1a and 1b.

**[Table 6] Body weight loss efficacy evaluation by 1-week repeated injection of acylated oxyntomodulin peptide of present invention**

| **Group** | **Drug and dose administered** | **Method of administration** | |
|---|---|---|---|
| Control | 0.1% Cremophor EL, vehicle | S.C. | once a day x 7 |
| Experimental group | Compound 1, 30 nmol/kg/QD | | |
| | Compound 2, 30 nmol/kg/QD | | |
| | Compound 7, 30 nmol/kg/QD | | |

From the experiment results, significant weight changes of -44.9%, -26.9% and - 6.8% from the initial body weight were observed in Compound 1, Compound 2 and Compound 7, respectively, which are acylated oxyntomodulin peptide analogs of the present invention; and cumulative feed consumption was reduced by 75%, 46% and 10%, respectively, compared to the vehicle control group.

### <Experimental Example 3> Body weight loss efficacy evaluation by 1-week repeated injection of the oxyntomodulin peptide analog of the present invention

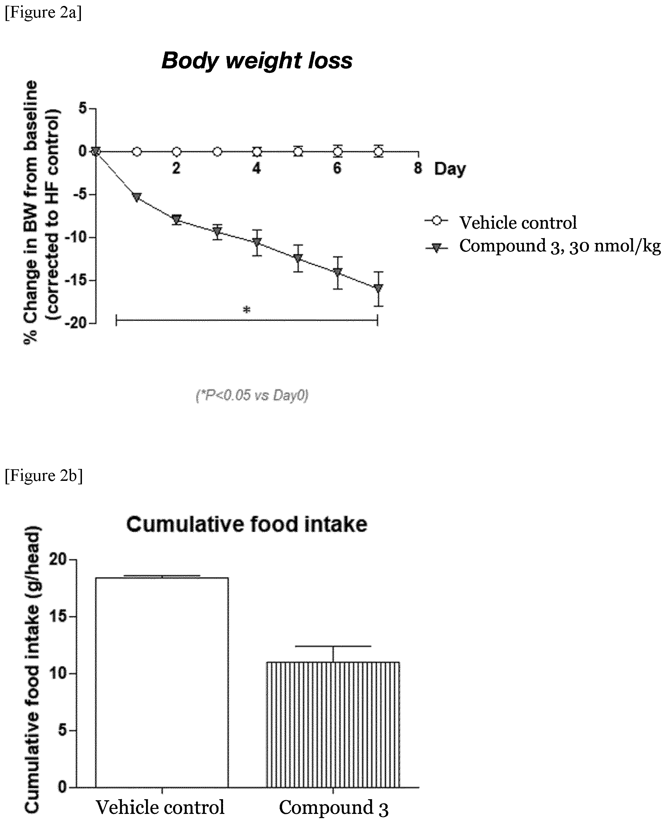

This experiment was conducted to confirm the weight loss efficacy of the acylated oxyntomodulin peptide analog according to the present invention. Male laboratory mice (C57BL/6 mouse) were provided with diet containing high fat. The mice with high-fat-diet-induced obesity were separated into groups by body weight before the experiment began. Compound 3, which is an acylated oxyntomodulin peptide analog according to an embodiment of the present invention, was prepared in sterile distilled water for injection containing 0.1% Cremophor EL to a dose of 30 nmol/kg. It was injected subcutaneously once a day for a total of 7 days as described in Table 9. Body weight and feed intake were measured once daily at the same time each day, to evaluate the body weight loss efficacy over time compared to the initial body weight, and the results are shown in Figures 2a and 2b.

**[Table 7] Body weight loss efficacy evaluation by 1-week repeated injection of acylated oxyntomodulin peptide of present invention**

| **Group** | **Drug and dose administered** | **Method of administration** | |
|---|---|---|---|
| Control | 0.1% Cremophor EL, vehicle | S.C. | once a day x 7 |
| Experimental group | Compound 3, 30 nmol/kg/QD | | |

From the experiment results, a significant change was observed in the body weight of the group injected with Compound 3, which is an acylated oxyntomodulin peptide analog according to the present invention, by -16.1% compared to the initial value, and the cumulative feed consumption was reduced by 40% compared to the vehicle control group.

### < Experimental Example 4 > Body weight loss efficacy evaluation by 1-week repeated injection of the oxyntomodulin peptide analog of the present invention

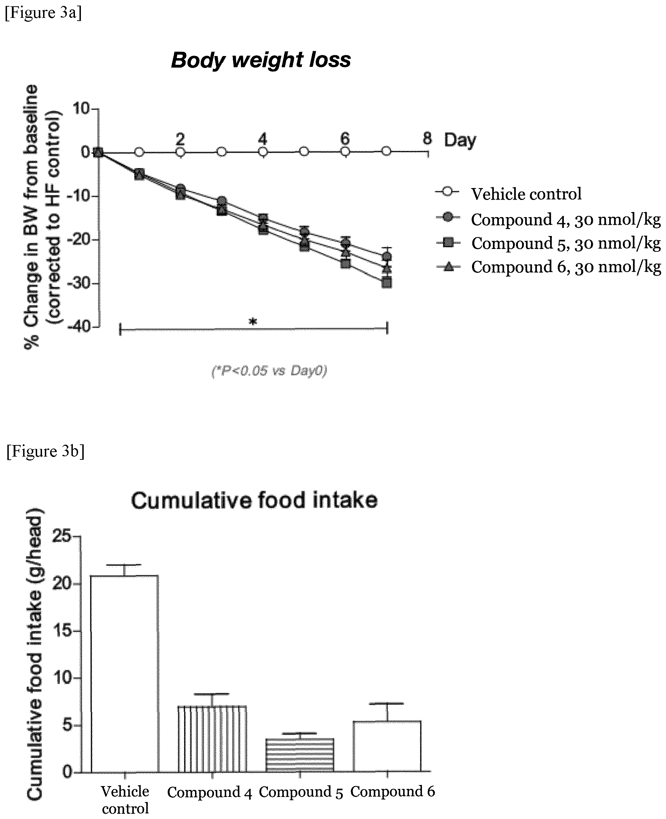

This experiment was conducted to confirm the weight loss efficacy of the acylated oxyntomodulin peptide analog according to the present invention. Male laboratory mice (C57BL/6 mouse) were provided with diet containing high fat. The mice with high-fat-diet-induced obesity were separated into groups by body weight before the experiment began. Compound 4, Compound 5, or Compound 6, which are acylated oxyntomodulin peptide analogs according to an embodiment of the present invention, was prepared in sterile distilled water for injection containing 0.1% Cremophor EL to a dose of 30 nmol/kg. It was injected subcutaneously once a day for a total of 7 days as described in Table 8. Body weight and feed intake were measured once daily at the same time each day, to evaluate the body weight loss efficacy of the acylated oxyntomodulin peptide analogs over time compared to the initial body weight, and the results are shown in Figures 3a and 3b.

**[Table 8] Body weight loss efficacy evaluation by 1-week repeated injection of acylated oxyntomodulin peptide of present invention**

| **Group** | **Drug and dose administered** | **Method of administration** | |
|---|---|---|---|
| Control | 0.1% Cremophor EL, vehicle | S.C. | once a day x 7 |
| Experimental group | Compound 4, 30 nmol/kg/QD | | |
| | Compound 5, 30 nmol/kg/QD | | |
| | Compound 6, 30 nmol/kg/QD | | |

From the experiment results, significant weight changes of -24.3%, -30.3% and - 26.9% of the initial body weight were observed with Compound 4, Compound 5 and Compound 6, respectively, which are acylated oxyntomodulin peptide analogs of the present invention, and cumulative feed consumption was reduced by 67%, 83%, and 75%, respectively, compared to the vehicle control group.

### < Experimental Example 5 > Assessment of Single-Dose Pharmacokinetic Profile in Mice

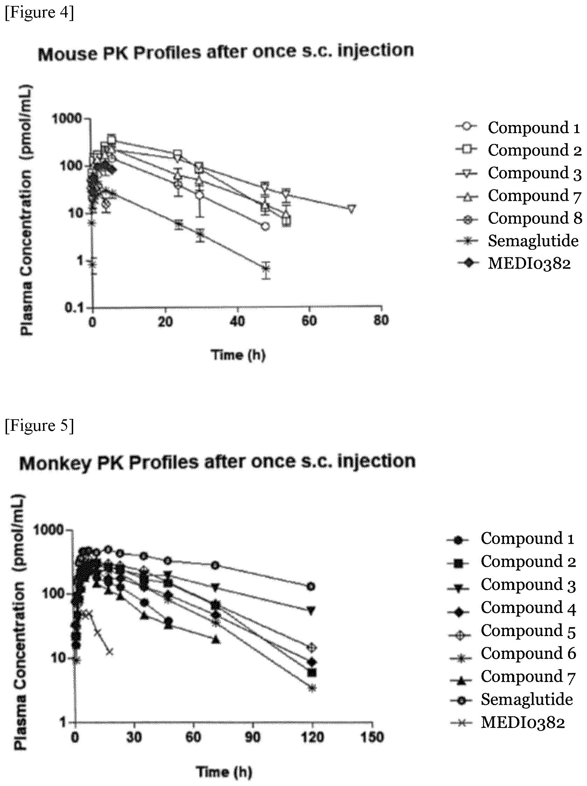

ICR mice were used to evaluate the pharmacokinetics in mice of the acylated oxyntomodulin peptide analogs according to the present invention. Compound 1, Compound 2, Compound 3, or Compound 7, which is an acylated oxyntomodulin peptide analog prepared by the present invention, was prepared in sterile distilled water for injection containing 0.1% Cremophore EL to a dose of 30 nmmol/kg, and injected to the ICR mice subcutaneously; then, blood was collected at a designated time after administration, and pharmacokinetic indicators were calculated through plasma drug concentration analysis, and the results are shown in Table 9 and Figure 4.

**[Table 9] Evaluation of pharmacokinetics in mice of acylated oxyntomodulin peptide analogs**

| | **Compound** | **Mouse PK (S.C. half-life)*** |
|---|---|---|
| Example | Compound 1 | B |
| | Compound 2 | B |
| | Compound 3 | B |
| | Compound 7 | B |
| Comparative Example | Compound 8 | A |
| | Semaglutide | B |
| | MEDI0382 | A |

| | | |
|---|---|---|
| ^{∗} A: Less than 6 hours, B: 6 hours or more | | |

As seen in Table 9 and Figure 4, the experimental results showed that the four acylated oxyntomodulin peptide analogs (Compound 1, Compound 2, Compound 3, Compound 7) according to the examples of the present invention exhibited a longer half-life of more than 6 hours compared to Comparative Example Compound 8 (Korea Patent Application No. 2018-0095717, Compound 3) and Comparative Example MEDI0382. It can be seen that the improved chemical stability due to the introduction of a novel albumin ligand leads to the significantly improved half-life compared to a single acylated oxyntomodulin peptide analog Comparative Example Compound 8.

Moreover, the half-life of Comparative Example Compound 8 was similar to the half-life of Comparative Example MEDI0382 under development as a once-a-day injection formulation, indicating that Comparative Example Compound 8 also can be developed only as a once-a-day injection formulation. On the other hand, the four acylated oxyntomodulin peptide analogs according to embodiments of the present invention showed improved PK half-life than Comparative Example Compound 8, and showed a half-life similar to Semaglutide developed as a once-weekly injection, indicating that the acylated oxyntomodulin peptide analog of the present invention can be developed as a long-acting drug in a once-weekly formulation.

### < Experimental Example 6 > Assessment of Single-Dose Pharmacokinetic Profile in Monkeys

Cynomolgus monkeys were used to evaluate the pharmacokinetics in monkeys of the acylated oxyntomodulin peptide analogs according to the present invention. Compound 1, Compound 2, Compound 3, Compound 4, Compound 5, Compound 6, or Compound 7, which is an acylated oxyntomodulin peptide analog prepared by the present invention, was prepared in sterile distilled water for injection containing 0.1% Cremophore EL to a dose of 100 nmmol/kg, and injected to the Cynomolgus monkeys subcutaneously once; then, blood was collected at a designated time after administration, and pharmacokinetic indicators were calculated through plasma drug concentration analysis. The results are shown in Table 10 and Figure 5.

**[Table 10] Pharmacokinetic evaluation in monkeys of acylated oxyntomodulin peptide analogs**

| | **Compound** | **Monkey PK (S.C. half-life)*** |
|---|---|---|
| Example | Compound 1 | C |
| | Compound 2 | C |
| | Compound 3 | E |
| | Compound 4 | D |
| | Compound 5 | D |
| | Compound 6 | C |
| | Compound 7 | E |
| Comparative Example | Compound 9 | B |
| | Compound 10 | A |
| | Compound 11 | B |
| | Compound 12 | B |
| | Semaglutide | E |
| | MEDI0382 | A |

| | | |
|---|---|---|
| * A: Less than 6 hours, B: 6 hours to less than 12 hours; C: 12 hours to less than 18 hours; D: 18 hours to less than 24 hours; E: 24 hours or more | | |

As seen in Table 10 and Figure 5 , the experimental results show that the seven acylated oxyntomodulin peptide analogs (Compounds 1 to 7) according to examples of the present invention exhibit a longer half-life of 12 hours or more compared to the four Comparative Example Compounds and Comparative Example MEDI0382 under development as an once-daily injection, indicating that the half-life was significantly improved through chemical stability improvement from the introduction of a new albumin ligand. In particular, Compound 3 and Compound 7 show a half-life similar to Semaglutide, which was developed as a once-weekly injection, with a monkey PK half-life of more than 24 hours, indicating that they can be developed as a long-acting drug in a once-weekly formulation.

Comparative Example Compound 9 is Comparative Example Compound 8 (Korea Patent Application No. 2018-0095717, Compound 3) except with an albumin ligand referenced in the literature (Nat. Commun. 2017;8: 16092), and had a half-life of less than 12 hours, suggesting that long-acting peptide analogs cannot be developed only with the introduction of the albumin ligand of the literature.

Comparative Example Compound 10, Comparative Example Compound 11, and Compound 7, having a lipophilic lipid in the end form of an aminocarboxyl group on the Lys side chain at position 34 of the peptide analog backbone, each have a carbon chain length of C14, C16, and C18, respectively. Compound 7 had a half-life of 24 hours or longer, whereas Comparative Example Compound 10 and Comparative Example Compound 11 showed less than 12 hours, indicating that when the lipophilic lipid ends with an aminocarboxyl group, it has a long half-life only when the carbon chain length is C18.

Compound 1, Compound 2, and Compound 3, each having a lipophilic lipid in the end form of a carboxyl group on the Lys side chain at position 34 of the peptide analog backbone, each have a carbon chain length of C14, C16, and C18, respectively. All of these substances showed a half-life of more than 12 hours, and in particular, Compound 3 showed a half-life of more than 24 hours. In order to have a long half-life, it is preferable to have a carboxyl group substituent at the end of the lipophilic lipid, and the longer the carbon chain, the better the effect.

Compounds 4, 5, and 6, in which a lipophilic lipid is bound to the Lys side chain at position 34 of the peptide analog backbone by a combination of a spacer and a polymeric moiety, have a longer half-life of 12 hours to less than 24 hours compared to the Comparative Examples. On the other hand, Comparative Example 12 showed a shorter half-life of less than 12 hours despite the lipophilic lipid linked to the polymeric moiety, suggesting that the effect of the end of the lipophilic lipid is greater than the effect of the polymeric moiety on the half-life.

### <Experimental Example 7> Testing the glucose tolerance improvement effect in mice

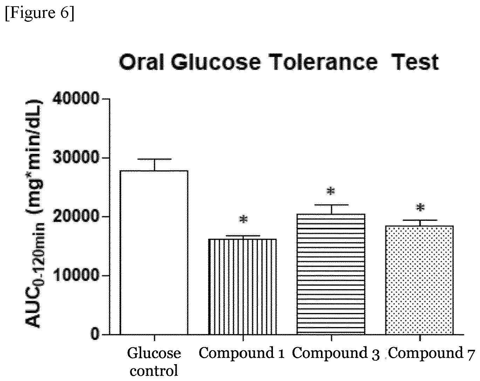

In this experiment, glucose tolerance improvement effect in male laboratory mice (ICR mice) of acylated oxyntomodulin peptide analogs according to the present invention was evaluated as improvement of postprandial glycemic control. Laboratory mice were fasted the day before the experiment. Then, Compound 2 or 3 or 7 according to the present invention was prepared in sterile distilled water for injection containing 0.1% Cremophor EL and injected subcutaneously 6 hours before glucose loading. Glucose solution was orally administered 6 hours after the injection of oxyntomodulin peptide analog. Whole blood glucose was measured via tail vein immediately before administering the drug and glucose, and for 2 hours after glucose loading at designated times. From the results, the area under the curve (AUC) of the blood glucose curve over time was produced to calculate the ratio of blood glucose AUC of the acylated oxyntomodulin peptide analog against the vehicle control as percentages. The results are shown in Figure 6.

As seen in Figure 6, Compound 2, Compound 3, or Compound 7, which is an acylated oxyntomodulin peptide analog according to the present invention, showed significant reduction of blood glucose AUC of 42.1%, 26.7%, and 33.9%, respectively, compared to the glucose control group, at 30 nmol/kg. In addition to the half-life improvement due to chemical stability, the acylated oxyntomodulin peptide analogs of the present invention are effective at improving blood glucose level and weight loss through activity on GLP-1 and glucagon receptors.

## Claims

1. An acylated oxyntomodulin peptide analog of the following Chemical Formula I: 
His-Aib-Gln-Gly-Thr-Phe-Thr-Ser-Asp-X1-Ser-Lys-Tyr-Leu-Asp-Aib-Arg-Arg-Ala-Gln-
Asp-Phe-Val-Gln-Trp-Leu-Met-Asn-Thr-Lys-Glu-Tyr-Glu-X2-Glu-Tyr-Glu (SEQ ID NO.14)
 wherein:
X₁ is functionalized Lys with [(2-(2-(2-aminoethoxy)ethoxy)acetoyl)2]-[gammaglutamyl]-[octadecanoyl] conjugated to its side chain;
X₂ is functionalized Lys with a lipophilic lipid or spacer-lipophilic lipid or polymeric moiety-spacer-lipophilic lipid or spacer-polymeric moiety-spacer-lipophilic lipid conjugated to its side chain;
the lipophilic lipid is of the following Structural Formula (1) or (2), 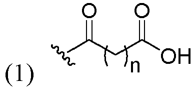 n = 12, 14 or 16 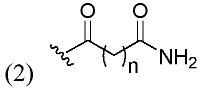 n = 16
the polymeric moiety is 1-3 (one or two or three) of 2-(2-(2-aminoethoxy)ethoxy)acetoyl; and
the spacer is r-Glu or Lys.

2. The acylated oxyntomodulin peptide analog according to claim 1, wherein the spacer is Lys, and the amine group of the amino acid residue of Lys spacer is covalently bonded to the lipophilic lipid or polymeric moiety.

3. The acylated oxyntomodulin peptide analog according to claim 1, wherein the spacer is r-Glu, and the a-amino group of the r-Glu spacer is covalently bonded to the lipophilic lipid or the polymeric moiety.

4. The acylated oxyntomodulin peptide analog of claim 1, wherein the acylated oxyntomodulin peptide analog is Compound 1 (SEQ ID NO. 2), Compound 2 (SEQ ID NO. 3), Compound 3 (SEQ ID NO. 4), Compound 4 (SEQ ID NO. 5), Compound 5 (SEQ ID NO. 6), Compound 6 (SEQ ID NO. 7) or Compound 7 (SEQ ID NO. 8).

5. A pharmaceutical composition for the prevention or treatment of a condition **characterized by** or caused by obesity or overweight, comprising the oxyntomodulin peptide analog of Chemical Formula I according to claim 1 as an active ingredient, and comprising a pharmaceutically acceptable excipient.

6. The pharmaceutical composition according to claim 5, wherein the composition is used in the form of an injection formulation.

7. A pharmaceutical composition for the treatment or prevention of non-insulin-dependent diabetes mellitus accompanied by obesity or overweight, comprising the oxyntomodulin peptide analog of Chemical Formula I according to claim 1 as an active ingredient, and comprising a pharmaceutically acceptable excipient.

8. The pharmaceutical composition according to claim 7, wherein the composition is used in the form of an injection formulation.
